# EUROPEAN PATENT SPECIFICATION

(11) **EP 1 253 913 B1**
(45) Date of publication and mention of the grant of the patent: **08.10.2003**
(21) Application number: 01911540.1
(22) Date of filing: 26.01.2001
(51) Int. Cl.: A61K 9/70

(54) **PATCH FOR LOCAL AND TRANSDERMAL ADMINISTRATION OF ACTIVE INGREDIENTS CONTAINING ANIONIC OR ELECTRON-ATTRACTING GROUPS**
PFLASTER FÜR DIE LOKALE UND TRANSDERMALE VERABREICHUNG VON WIRKSTOFFEN MIT ANIONISCHEN ODER ELEKTRONEN-ANZIEHENDEN GRUPPEN
TIMBRE DESTINE A L'ADMINISTRATION TRANSDERMIQUE ET LOCALE DE PRINCIPES ACTIFS CONTENANT DES GROUPES ANIONIQUES OU ELECTROATTRACTEURS

(30) Priority: 26.01.2000 IT MI20000095
(43) Date of publication of application: 06.11.2002
(73) Proprietor: F.T. Holding S.A., 1150 Luxembourg (LU)
(72) Inventor: ROVERSI, Francesco, CH-6900 Lugano (CH); CILURZO, Francesco, I-20154 Milano (IT)
(74) Representative: Gervasi, Gemma, Dr.
(86) International application number: EP0100870
(87) International publication number: WO01054674

(56) References cited:
- WO-A-97/39741
- US-A- 5 456 745
- DATABASE WPI Section Ch, Week 199244 Derwent Publications Ltd., London, GB; Class A96, AN 1992-363069 XP002170006 -& JP 04 266818 A (SEKISUI CHEM IND CO LTD), 22 September 1992 (1992-09-22)
- VENKATRAMAN S ET AL: "Skin adhesives and skin adhesion - 1. Transdermal drug delivery systems" BIOMATERIALS,GB,ELSEVIER SCIENCE PUBLISHERS BV., BARKING, vol. 19, no. 13, June 1998 (1998-06), pages 1119-1136, XP004161374 ISSN: 0142-9612
- LIN SHAN-YANG ET AL: "The effect of plasticizers on compatibility, mechaincal properties, and adhesion strength of drug-free Eudragit E films" PHARMACEUTICAL RESEARCH,US,NEW YORK, NY, vol. 8, no. 9, September 1991 (1991-09), pages 1137-1143, XP002107579 ISSN: 0724-8741

## Description

A patch for administering by local and transdermal route active ingredients containing anionic or electron-attracting groups, makes it possible to obtain effective concentrations of said active ingredients and is easy to use.

### STATE OF THE ART

Patches are useful both for transdermal administration and for local administration of active principles.

Administration by transdermal route has over the years become increasingly appreciated, in that it enables effective blood levels to be obtained for more prolonged intervals of time.

As compared to what occurs in the case of creams and ointments, local administration made with patches enables precise doses of the active principle, its controlled release, as well as the delimitation and protection of the area of administration.

The problems connected to this type of administration regard both the stability of the active ingredient and its capacity for being effectively released by the pharmaceutical form. On the other hand, the said pharmaceutical form must be manageable for use, i.e., provided with good adhesiveness and detachable both from the protective film and from the skin without leaving any residue.

US 5, 296,512 (in the name of Rohm GmbH Chemische Fabrik) describes transdermal pharmaceutical forms which are removable with water and consist of a backing layer coated with mono-ethylenically unsaturated mono-carboxylic acid or di-carboxylic acid and of at least one ester of (meth)acrylic acid copolymer. This pharmaceutical form presents, however, drawbacks of a cohesive nature such as to be prejudicial to their entry on the market. The aqueous polymeric systems, like that disclosed in this patent, have been until now proposed by the manufacturers, mainly to make the adhesive layer in bilayered patches; their use in order to achieve controlled drug release is poorly investigated also because of their low cohesive properties. It is therefore interesting to improve the technological characteristic of the dry matrix, such as adhesive properties and drug release, by adding to the polymeric mixture other excipients, enhancing the cohesive properties of said polymeric systems. For this purpose the most conventional approach is to introduce, into the polymeric system, bivalent ions, such as zinc, usually in the form of zinc sulphate, which act as cross-linking agents. However, the addition of even small quantities of zinc sulphate causes aggregation of the polymeric chains on account of the strong interaction between the carboxyl groups of the polymer and the ion.

A second approach to solving the above problem is the addition of a second polymer to the matrix, but in view of the plethora of compounds that may be used in this way (polycarboxymethylenes, hydroxymethyl celluloses, hydroxypropyl celluloses, carboxymethyl celluloses, arabic gum, Tragacanth or guar gum, polyvinyl pyrrolidones, etc.), the solution of the problem is far from immediate also because, In addition to the problems connected to the compatibility with the other polymers present and the characteristics of adhesiveness and cohesion sought, it is necessary to take into account the compatibility of this new component with the active principles.

USP 5,456,745 discloses a flexible hydrophylic, water swellable but insoluble gel film containing contemporaneously cationic copolymers and anionic copolymers, containing as optional additive with the function of thickener polyvinylpyrrolidone.

WO97/39741 discloses device for transdermal administration of N-acetyl-L-cysteine optionally encompassing salts, prodrugs and metabolites thereof optionally together. Among the possible transdermal devices mention is made of the following two types of devices the drug In adhesive type and the multilaminate type, similar to the drug in adhesive, but which Incorporates an additional layer of pressure sensitive adhesive to cover the entire device and affix to the skin. This adhesive layer is made of a cationic polyacrylate containing ammonium groups.

In JP 04266818 is disclosed an adhesive matrix containing a blend of weakly basic organic carboxylate the obtained adhesive can stick for a long time without peeling, however the matrix therein disclosed is an alcoholic matrix. Moreover the polyvinylpyrrolidone used in D₃ has a molecular weight up to 54.000 Da.

### SUMMARY OF THE INVENTION

There has now been surprisingly found a patch for local and transdermal administration of active ingredients containing anionic or electron-attracting groups which is able to obtain effective concentrations of active ingredient and afford both good adhesiveness and an optimal detachment both from the protective film and from the skin.

Consequently, the present invention relates to a patch for local and transdermal administration of an active ingredient having at least one anionic or electron-attracting group and pharmaceutically acceptable salts thereof comprising:
a) a prevalently aqueous polymeric matrix containing said active ingredient, whose polymer is selected from the group consisting of:
   a-1) an anionic copolymer of methacrylic acid with a methacrylate ester with a C₁-C₁₀ linear or branched alcohol, or
   a-2) a cationic copolymer of methacrylic acid with a C₁-C₁₀ linear or branched alcohol containing a tertiary aminic group, and a neutral methacrylate ester with a C₁-C₁₀ linear or branched alcohol, said polymeric matrix further comprising
   a3) 30-100% by weight based on the copolymer weight of a polyvinylpyrrolidone having an average molecular weight ranging from 300.000 to 1.350.000 polyvinylpyrrolidone,
   a4) a plasticizer,
b) a backing layer which the matrix is adhered to,
c) a protective film removable at the moment placed on said polymeric matrix (a).

### DESCRIPTION OF THE FIGURES

Figure 1 shows the *in vitro* permeation graph for Formulation 2 of Example 1 containing Flurbiprofen as compared to the commercial product Transact™.
Figure 2 shows the *in vitro* permeation graph for Formulation 1 of Example 1 containing Ketoprofen as compared to the commercial product Fastum Gel™.
Figure 3 shows the *in vitro* permeation graph for Formulation 3 containing sodium Diclofenac of Example 3 as compared to the commercial product Dicloreum Tissugel™.

In ordinates of said Figures reported is the amount of permeated active ingredient expressed as µg/cm², and in abscissae reported is the time expressed in hour.

### DETAILED DESCRIPTION OF THE INVENTION

The anionic copolymer of type (a1) or cationic copolymer of type (a2) have preferably a molecular weight ranging from 80,000 to 500,000, more preferably from 100,000 to 300,000.

According a preferred embodiment the copolymer (a1) is selected from the copolymer of methacrylic acid and methylmethacrylate and the copolymer of methacrylic add and ethylmethacrylate, whereas the cationic copolymer (a2) is selected from the copolymers of dimethylaminoethylmethacrylate with methylmethacrylate and dimethylaminoethylmethacrylate with butylmethacrylate, and mixtures thereof.

According to a more preferred embodiment of the present invention the specific product already available on the market with the commercial name EUDRAGIT™ L, and specifically EUDRAGIT™ L100, which is in the form of a white fine powder, is used as the methacrylic acid -methylmethacrylate copolymer belonging to the aforementioned class (a1). This product is characterised by having a ratio of free carboxylic groups/ester groups being approximately 1:1 and an average molecular weight of 135000. Analogously according to a more preferred embodiment, the product sold with the commercial name EUDRAGIT ™ L-100-55, and in the form of a white fine powder is used as the methacrylic acid -ethylmethacrylate copolymer belonging to class (a1). This product is characterised by having the ratio of free carboxylic acid/ester groups is about 1/1. Said product has an average molecular weight of 250,000. More preferably a mixture of copolymer dimethylaminoethylmethacrylate - methylmethacrylate and copolymer dimethylaminoethylmethacrylate - butylmethacrylate is used as the polymeric cation belonging to class (a2) has an average molecular weight of 150,000 and is available on the market with the trademark EUDRAGIT ™E, and in particular EUDRAGIT™ E100, which is in the form of white to yellow tinged granules.

For high molecular weight polyvinylpyrrolidone we mean a polyvinylpyrrolidone having an average molecular weight ranging from 300,000 to 1,350,000.

According to particularly preferred embodiments the product available on the market with the commercial name: KOLLIDON 90™ F sold by BASF and characterised by having an average molecular weight of 1,100,000, or the product PVP33000 sold by Carlo Erba having an average molecular weight of 335,000 is used as polyvinylpyrrolidone.

Examples of plasticizing agents useful for the purposes of the present invention are acetyl tributyl citrate, tributyl citrate, glycerine, propylene glycol, polyethylene glycols (PEGs) having various molecular weights, phthalates and triethyl citrate. Optionally, the patch of the present invention may contain non-ionic and anionic surfactants.

The patch of the present invention is suitable for vehicling active ingredients containing anionic groups or electron attracting groups. These active ingredients belong preferably to the following therapeutical classes: non-steroidal antiinflammatory drugs, such as Ketoprofen, Flurbiprofen, Ibuprofen, Naproxen, Indomethacin, Diclofenac Piroxicam, and salts thereof, muscle relaxants such as Thiocolchicoside, antihypertensive drug such as dihydropyridine, and in particular Nifedipine, Clonidine, analgesics such as Fentanyl, and local anaesthetics such as Lidocaine, antianginal drugs such as Nitroglycerin.

The active ingredients contained in the matrix of the patch of the invention are able to be vehicled locally and transdermally *in vivo* with results at least comparable to those of other transdermal systems at present commercially available, as will be shown in what follows, whilst the cohesiveness of the matrix is altogether excellent.

Optionally, it is possible to add to the matrix of the patch of the invention enhancers without these adversely affecting the degree of cohesion of the matrix itself. Amongst the enhancers the following may be cited as examples: Transcutol (diethylen glycolmonoethylether), propylene glycol, polyhydroxylated castor oil, polyethylene glycols of different molecular weights, unsaturated and saturated acids and esters thereof such as isopropylmyristate, Lauroglycol (propylenglycol monolaurate or dilaurate), Labrafil (macrogol glyceride ester with oleic acid), Labrasol (macrogol glyceride with caprylic acid), polysorbates such as Tween®, and Span®and in particular Span®80, polyoxyethylenalcohols such as Brij®, and in particular Brij®58 (cetomacrogol 1000), and terpenes, such as limonene menthol, eucalyptol.

In the patch according to the backing layer (b) is preferably made of woven fabric, non-woven fabric, polymeric film and foaming material.

The patch according to the present invention may be prepared as follows.

A prevalently aqueous solution of the copolymers of the aforementioned class (a1) or (a2) is prepared . In case the copolymer of class (a1) is used containing free carboxyl groups, it is advisable, for the purpose of obtaining its dissolution, to add an aqueous solution of a hydroxide of an alkaline or alkaline-earth metal, preferably sodium hydroxide or potassium hydroxide. Once a homogeneous solution has been obtained, there is to be added, at least one plasticizing agent. Finally, a solution of polyvinylpyrrolidone (PVP) is added to the above solution. Then, after undergoing stirring the solution is left to stand to enable removal of any air that may be trapped in it.

The PVP Is used in concentrations ranging from 5 to 50 wt%, and in aqueous solution and/or in solution with an appropriate water-mixable organic solvent. All the other solutions useful for preparation of the matrix of the invention. Including the one containing the active principle, are water solutions, optionally containing up to a maximum of 45 wt% of a water-mixable organic solvent

The mixture thus obtained is spread and dried onto the protective film (c), and finally made to adhere to the backing layer (b).

The active ingredient In aqueous solution referred to above may be added to a solution of the copolymer (a1) or (a2), or alternatively to that of the PVP before the latter Is mixed with the solution of the polymer or copolymer. The alternative depends upon the characteristics of solubility of the active principle.

The temperature at which the entire process takes place ranges from 20°C to 80°C according to the type of polymer or copolymer used.

The plasticizing agent Is contained In an amount of 5-200 wt% with respect to the copolymer weight.

The surfactant is contained In an amount of 0.1-100 wt% with respect to the copolymer weight

The enhancers are contained in an amount of 1-150 wt% with respect to the polymer or copolymer.

The Invention will now be illustrated In greater detail by the following examples.

### EXAMPLE 1

### A. Formulation 1

| Composition of the matrix | |
|---|---|
| Ketoprofen | 1.9 g |
| Eudragit L | 10.7 g |
| Water | 16.4 g |
| Sodium hydroxide | 1.9 g |
| Water | 19.2 g |
| PEG 400 | 16.1 g |
| Glycerine | 7.0 g |
| Polyvinyl pyrrolidone 33000 (PVP) | 5.4 g |
| Water | 21.4 g |

### Process of fabrication

### Solution A

Eudragit L was dispersed in water under propeller stirring at a speed of 100 rpm for 10 minutes. Prepared apart was the solution of sodium hydroxide, which was added as fast as possible to the suspension of Eudragit L, and the speed of the stirrer was increased gradually up to 250 rpm. After approximately 5 minutes a homogeneous solution was obtained to which PEG 400 and glycerine were added. The resulting system was kept under stirring at the same speed for a further 15 minutes and then left to stand up to complete removal of any air that might have been entrapped therein.

### Solution B

The PVP was added slowly to the water heated to 50°C and kept under stirring with a magnetic stirrer. Any water that might have evaporated was restored after cooling to room temperature.

### Addition of the active principle

The active ingredient Ketoprofen was added to solution A, which had been heated to 40°C, and the system was kept under magnetic stirring up to complete dissolution of the Ketoprofen (approximately 15 minutes). At the end of the process the solution B previously heated to the same temperature was added, and the resulting dispersion was further stirred for 15 minutes. Any water that might have evaporated was restored. The resulting polymeric system was left to stand, up to complete removal of any air that might have been englobed therein, before being used.

### B. Formulation 2

The procedure was as for point A, with the same components and amounts, but replacing the Ketoprofen with 1.9 g of Flurbiprofen.

Formulations 1 and 2 were used for preparing patches according to the following specifications:

| | |
|---|---|
| Backing layer | Artificial silk |
| Protective film | siliconized paper |
| Spreading on the protective film and drying of the matrix were performed with a Matis spreading machine - Model: LTE-S | |
| Rate of spreading | 1m/min |
| Drying time | 15 min |
| Drying temperature | 60°C with horizontal air circulation |
| Distance between knife and protective film | 300 µm |
| At the end of the drying process, the matrix dried on siliconized paper was made to adhere to the backing layer. | |

### EXAMPLE 2

### Formulation 3

| Composition of the matrix | |
|---|---|
| Sodium Diclofenac | 1.8 g |
| Eudragit L | 10.35 g |
| Water | 15.9 g |
| Sodium hydroxide | 1.85 g |
| Water | 18.6 g |
| PEG 400 | 15.5 |
| Glycerine | 6.8 g |
| Polyvinyl pyrrolidone 33000 (PVP) | 5.2 g |
| Methanol | 20.6 g |
| Tween 80 | 0.6 g |
| Transcutol | 2.8 g |

### Process of fabrication

### Solution A

Eudragit L was dispersed in water under propeller stirring at a speed of 100 rpm for 10 minutes. Prepared apart was the solution of sodium hydroxide, which was added as fast as possible to the suspension of Eudragit L, and the speed of the stirrer was increased gradually up to 250 rpm. After obtaining a homogeneous solution, PEG 400 and glycerine were added. The resulting system was kept under stirring at the same speed for a further 15 minutes and then left to stand up to complete removal of any air that might have been englobed therein.

### Solution B

The PVP was added slowly to the methanol and kept under stirring with a magnetic stirrer up to complete solubilization. Any methanol that might have evaporated was restored. Next Tween 80 and Transcutol were added.

### Addition of the active principle

The active ingredient sodium Diclofenac was added to solution B, and the system was kept under magnetic stirring for 15 minutes. At the end of the process, solution A was added, and the resulting dispersion was further stirred for 15 minutes. Any water that might have evaporated was restored. The resulting polymeric system was left to stand, up to complete removal of any air that might have been englobed therein, before being used.

Formulation 3 was used for preparing a patch according to the following specifications:

| | |
|---|---|
| Backing layer | PVC |
| Protective film | siliconized paper |
| Spreading on the protective film and drying of the matrix were performed with a Matis spreading machine - Model LTE-S | |
| Rate of spreading | 1m/min |
| Drying time | 12 min |
| Drying temperature | 50°C |
| Distance between knife and protective film | 300 µm |
| At the end of the drying process, the matrix dried on the siliconized paper was made to adhere to the backing layer. | |

### EXAMPLE 3

Proceeding as described in Example 1, a series of formulations without, however, the active ingredient and having the following composition in grams was prepared:

| Formulation | Eudragit L | Glycerine | PEG | NaOH | PVP |
|---|---|---|---|---|---|
| 4 | 33.07 | 19.48 | 40.33 | 5.95 | 2.91 |
| 5 | 29.13 | 19.03 | 43.69 | 5.24 | 5.66 |
| 6 | 28.3 | 18.49 | 42.45 | 5.09 | 9.8 |
| 7 | 27.06 | 17.68 | 40.59 | 4.87 | 14.29 |

All four of the above formulations presented excellent characteristics of adhesiveness.

### EXAMPLE 4

### Formulation 8

| Composition of the matrix | |
|---|---|
| Eudragit E | 10.5 G |
| Water | 49.2 G |
| Lauric acid | 6.2 G |
| Adipic acid | 1.2 G |
| Glycerine | 6.9 G |
| Polyvinyl pyrrolidone 33000 (PVP) | 5.2 G |
| Water | 20.8 G |

### Process of fabrication

### Solution A

Eudragit E, lauric acid and adipic acid were dispersed in water at 80°C in vacuum conditions and under propeller stirring at a speed of 100 rpm for 60 minutes. The temperature was then brought down to 60°C, and glycerine was added. The resulting system was kept under stirring at the same speed for a further 15 minutes and then left to stand up to complete removal of any air that might have been entrapped.

### Solution B

The PVP was added slowly to the water preheated at 50° and kept under stirring with a magnetic stirrer up to complete solubilization. Any water that might have evaporated was restored.

Solutions A and B were mixed at room temperature with a propeller stirrer until a homogeneous solution was obtained.

Formulation 8 resulting from the above process presented excellent characteristics of adhesiveness.

### EXAMPLE 5

### Comparison formulations

Proceeding as described for Example 1, a series of comparison formulations. without the active ingredient and the polyvinylpyrrolidone, having the following compositions in grams were prepared:

| Formulation | Eudragit L | Glycerine | PEG | NaOH | CMC | HPMC | HMC | PA | GA |
|---|---|---|---|---|---|---|---|---|---|
| 9 | 30 | 19.6 | 45 | 5.4 | | | | 0.9 | |
| 10 | 36.98 | 22.19 | 33.28 | 6.66 | | | | | 0.8 |
| 11 | 26.79 | 17.5 | 40.18 | 4.82 | 10.71 | | | | |
| 12 | 26.1 | 17.05 | 39.14 | 4.7 | 13.01 | | | | |
| 13 | 26.01 | 16.99 | 39.01 | 4.687 | | 13.31 | | | |
| 14 | 27.15 | 17.74 | 40.72 | 4.89 | | 9.51 | | | |
| 15 | 29.13 | 19.03 | 43.69 | 5.24 | | 2.91 | | | |
| 16 | 26.01 | 16.99 | 39.01 | 4.68 | | | 13.31 | | |
| PEG = polyethylene glycol | | | | | | | | | |
| CMC = carboxymethyl cellulose | | | | | | | | | |
| HPMC = hydroxypropylmethyl cellulose | | | | | | | | | |
| HMC = hydroxymethyl cellulose | | | | | | | | | |
| PA = polyacrylate | | | | | | | | | |
| GA = gum arabic | | | | | | | | | |

None of the formulations 9-16 were found to possess satisfactory characteristics of adhesiveness; i.e., they were either too sticky and hence hard to work or else too stiff and hence not suitable for ensuring adhesion to the skin.

### EXAMPLE 6

The formulations referred to in Examples 1 and 2 underwent a skin-permeation test *in vitro* with human epidermis according to the procedure described in Minghetti P. *et al.,* J. Pharm. Pharmacol., 1999, 51:673-678, in comparison with commercially available products.

The results appear in the attached figures.

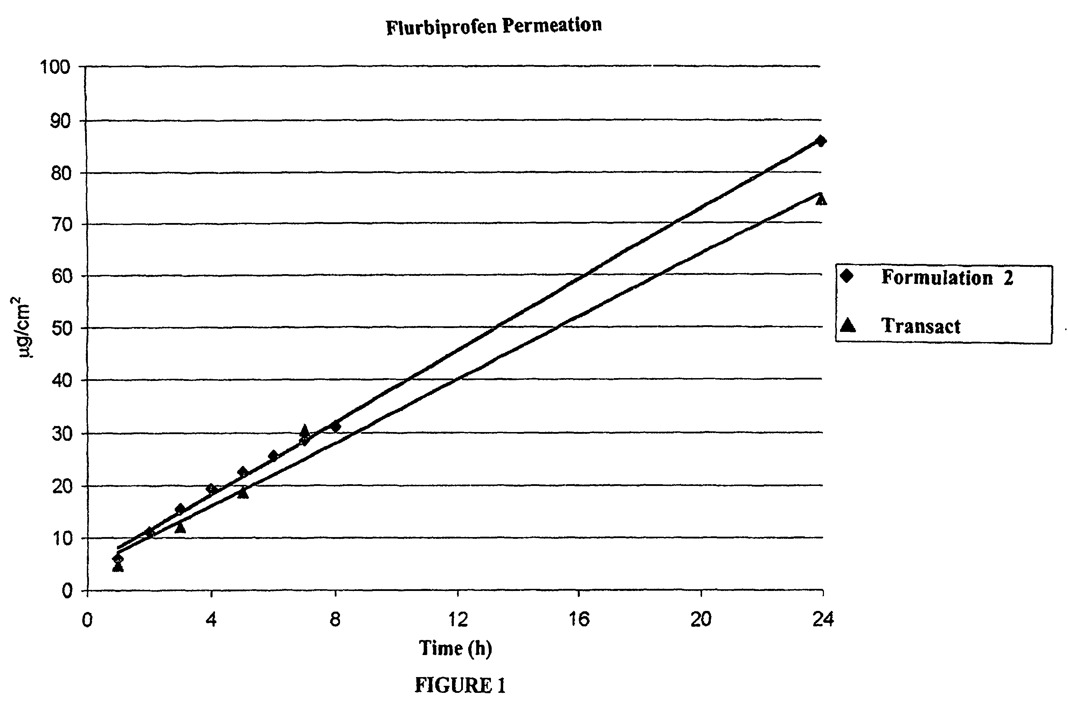

Figure 1 shows the permeation graph for Formulation 2 containing Flurbiprofen as compared to the commercial product Transact (The Boots Company). The straight lines indicating permeation for the two products are practically equivalent.

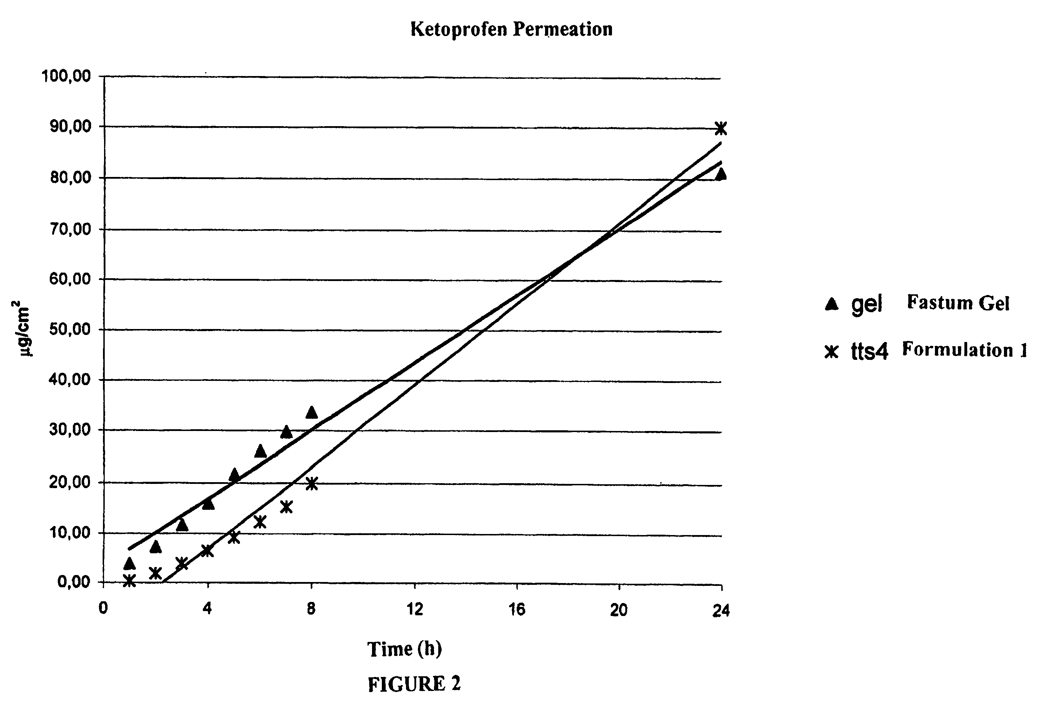

Figure 2 shows the permeation graph for Formulation 1 containing Ketoprofen as compared to the commercial product Fastum Gel (Menarini). The straight lines indicating permeation for the two products are practically equivalent.

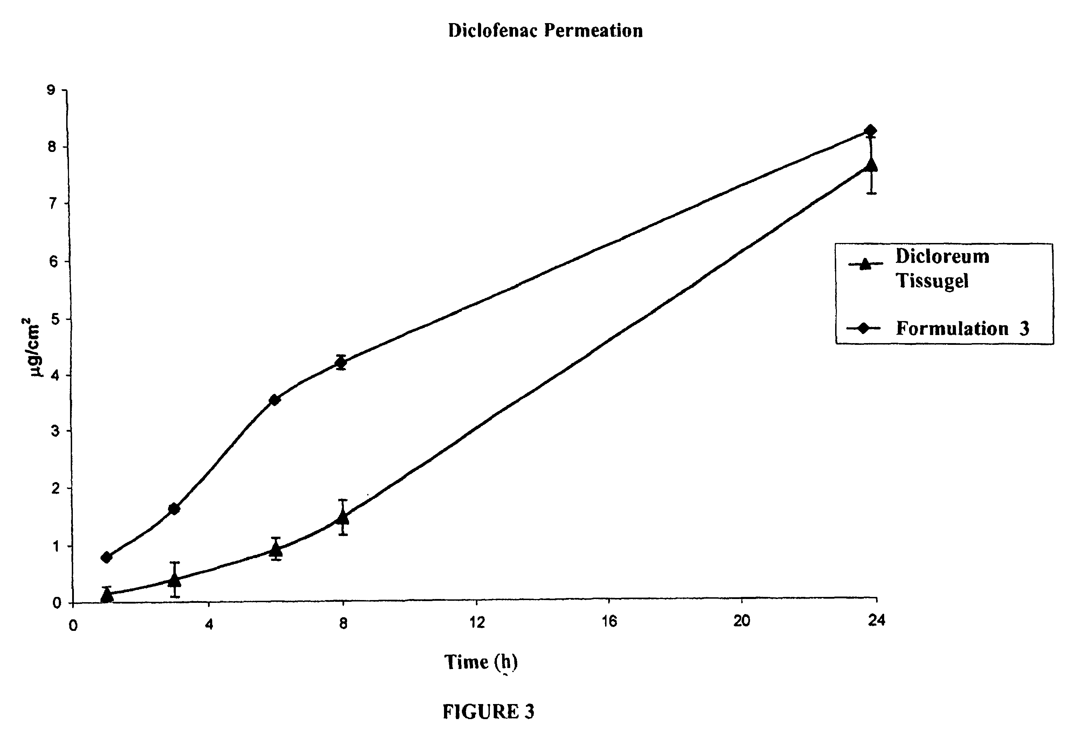

Figure 3 shows the permeation graph of Formulation 3 containing sodium Diclofenac as compared to the commercial product Dicloreum Tissugel (Alfa Wasserman). The straight lines indicating permeation for the two products are practically equivalent.

### EXAMPLE 7

| Composition of the polymeric systems used for the preparation of the matrices | | | | | | | | | |
|---|---|---|---|---|---|---|---|---|---|
| Components | Form. No. 1 | Form. No. 2 | Form. No. 3 | Form. No. 4 | Form. No. 5 | Form. No. 6 | Form. No. 7 | Form. No. 8 | Form. No. 9 |
| Eudragit L | 4.5 | 4.5 | 4.5 | 4.5 | 4.5 | 4.5 | 4.5 | 4.5 | 4.5 |
| Water | 6.9 | 6.9 | 6.9 | 6.9 | 6.9 | 6.9 | 6.9 | 6.9 | 6.9 |
| NaOH | 0.81 | 0.81 | 0.81 | 0.81 | 0.81 | 0.81 | 0.81 | 0.81 | 0.81 |
| Water | 6.61 | 9.6 | 11.95 | 7.99 | 10.34 | 5.87 | 8.19 | 4.26 | 8.1 |
| PEG 400 | 5.41 | 8.09 | 8.09 | 8.09 | 8.09 | 5.41 | 5.41 | 5.41 | 6.75 |
| Glycerine | 2.36 | 3.52 | 3.52 | 2.36 | 2.36 | 3.52 | 3.52 | 2.36 | 2.94 |
| PVP | 1.8 | 2.7 | 1.8 | 2.7 | 1.8 | 2.7 | 1.8 | 2.7 | 2.25 |
| Water | 7.2 | 10.8 | 7.2 | 10.8 | 7.2 | 10.8 | 7.2 | 10.8 | 9 |

### Preparation

### Solution A

Eudragit L 100 was dispersed in water under propeller stirring at a speed of 100 rpm for 10 minutes. A solution of sodium hydroxide was prepared apart.

The solution of sodium hydroxide was added as fast as possible to the suspension of Eudragit L 100, and the speed of the stirrer was increased gradually up to 200 rpm. After a homogeneous solution had been obtained (approximately 5 minutes), PEG 400 and glycerine were added. The resulting system was kept under stirring at the same speed for a further 15 minutes and then left to stand, up to complete removal of any air that might have been entrapped therein.

### Solution B

The PVP was added very slowly to the water preheated at 50°C and kept under stirring with a mechanical stirrer up to complete solubilization. Any water that might have evaporated was restored.

Solution B was added to solution A, and the dispersion obtained was kept under mechanical stirring for a further 30 minutes.

The resulting polymeric system was left to stand, up to complete removal of any air that might have been englobed therein, before being used.

| | |
|---|---|
| Backing layer | artificial silk |
| Protective film | siliconized paper |

Spreading on thebacking layer and drying of the matrix were performed with a Matis spreading machine - Model: LTE-S

| | |
|---|---|
| Rate of spreading | 1m/min |
| Drying time | 15 min |
| Drying temperature | 50°C |
| Distance between knife and protective film | 300 µm |

At the end of the drying process, the matrix dried on thebacking layer was made to adhere to the protective film.

### EXAMPLE 8

| Composition of the polymeric systems used for the preparation of the matrices | | | | |
|---|---|---|---|---|
| | Form. No. 2 | Form. No. 3 | Form. No. 4 | Form. No. 5 |
| Indomethacin | 1.9 | = | = | = |
| Ibuprofen | = | 1.9 | = | = |
| Naproxen | = | = | 1.9 | = |
| Pyroxicam | = | = | = | 1.9 |
| Eudragit L100 | 10.7 | 10.7 | 10.7 | 10.7 |
| Water | 16.4 | 16.4 | 16.4 | 16.4 |
| Sodium hydroxide | 1.9 | 1.9 | 1.9 | 1.9 |
| Water | 19.2 | 19.2 | 19.2 | 19.2 |
| PEG 400 | 16.1 | 16.1 | 16.1 | 16.1 |
| Glycerine | 7.0 | 7.0 | 7.0 | 7.0 |
| PVP | 5.4 | 5.4 | 5.4 | 5.4 |
| Water | 21.4 | 21.4 | 21.4 | 21.4 |

### Preparation

### Solution A

Eudragit L 100 was dispersed in water under mechanical stirring at a speed of 100 rpm for 10 minutes. A solution of sodium hydroxide was prepared apart.

The solution of sodium hydroxide was added as fast as possible to the suspension of Eudragit L 100, and the speed of the stirrer was increased gradually up to 200 rpm. After a homogeneous solution had been obtained (approximately 5 minutes), PEG 400 and glycerine were added. The resulting system was kept under stirring at the same speed for a further 15 minutes and then left to stand, up to complete removal of any air that might have been entrapped therein.

### Solution B

The PVP was added very slowly to the water preheated at 50°C and kept under stirring with a mechanical stirrer up to complete solubilization. Any water that might have evaporated was restored.

The active ingredient was added slowly to solution A, and the mixture was kept under stirring until a homogeneous solution was obtained. At the end of the process, solution B was added, and the dispersion obtained was stirred for a further 30 minutes.

The resulting polymeric system was left to stand, up to complete removal of any air that might have been englobed therein, before being used.

| | |
|---|---|
| Backing layer | PVC or artificial silk or non-woven cotton fabric |
| Protective film | siliconized paper |

Spreading on the backing layer and drying of the matrix were performed with a Matis spreading machine - Model: LTE-S

| | |
|---|---|
| Rate of spreading | 1m/min |
| Drying time | 15 min |
| Drying temperature | 60°C |
| Distance between knife and protective film | 300 µm |

At the end of the drying process, the matrix dried on thebacking layer was made to adhere to the protective film.

### EXAMPLE 9

| Composition of the polymeric system used for the preparation of the matrix | |
|---|---|
| Pyroxicam | 0.5 |
| Eudragit L | 2.25 |
| Water | 3.45 |
| Sodium hydroxide | 0.405 |
| Water | 4.05 |
| PEG 400 | 3.375 |
| Glycerine | 1.47 |
| PVP | 1.41 |
| Methanol | 7.99 |
| Transcutol | 2 |
| Polyhydroxylated castor oil | 1 |
| PEG 400 | 0.9 |
| Tween 80 | 0.4 |
| Eucalyptus oil | 0.23 |

### Preparation

### Solution A

Eudragit L 100 was dispersed in water under mechanical stirring at a speed of 100 rpm for 10 minutes. A solution of sodium hydroxide was prepared apart.

The solution of sodium hydroxide was added as fast as possible to the suspension of Eudragit L 100, and the speed of the stirrer was increased gradually up to 200 rpm. After a homogeneous solution had been obtained (approximately 5 minutes), PEG 400 and glycerine were added. The resulting system was kept under stirring at the same speed for a further 15 minutes and then left to stand, up to complete removal of any air that might have been entrapped therein.

### Solution B

The PVP was added very slowly to the methanol and kept under stirring with a mechanical stirrer up to complete solubilization. Any methanol that might have evaporated was restored.

The PEG 400, Transcutol, polyhydroxylated castor oil, Tween 80, and active ingredient were added slowly to solution B, and the mixture was kept under stirring until a homogeneous solution was obtained. At the end of the process, solution A and the eucalyptus oil were added, and the dispersion obtained was stirred for a further 30 minutes.

The resulting polymeric system was left to stand, up to complete removal of any air that might have been englobed therein, before being used.

| | |
|---|---|
| Backing layer | PVC |
| Protective film | siliconized paper |

Spreading on the backing layer and drying of the matrix were performed with a Matis spreading machine - Model: LTE-S

| | |
|---|---|
| Rate of spreading | 1m/min |
| Drying time | 15 min |
| Drying temperature | 50°C |
| Distance between knife and protective film | 300 µm |

At the end of the drying process, the matrix dried on the backing layer was made to adhere to the protective film.

### EXAMPLE 10

| Composition of the polymeric system used for the preparation of the matrix | |
|---|---|
| Naproxen | 0.2 |
| Eudragit L | 1.07 |
| Water | 1.64 |
| Sodium hydroxide | 0.19 |
| Water | 1.92 |
| PEG 400 | 1.6 |
| Glycerine | 0.7 |
| PVP | 0.54 |
| Methanol | 2.16 |
| PEG 400 | 0.5 |
| Tween 80 | 0.1 |

### Preparation

### Solution A

Eudragit L 100 was dispersed in water under propeller stirring at a speed of 100 rpm for 10 minutes. A solution of sodium hydroxide was prepared apart.

The solution of sodium hydroxide was added as fast as possible to the suspension of Eudragit L 100, and the speed of the stirrer was increased gradually up to 200 rpm. After a homogeneous solution had been obtained (approximately 5 minutes), PEG 400 and glycerine were added. The resulting system was kept under stirring at the same speed for a further 15 minutes and then left to stand, up to complete removal of any air that might have been entrapped therein.

### Solution B

The PVP was added very slowly to the methanol and kept under stirring with a mechanical stirrer up to complete solubilization. Any methanol that might have evaporated was restored.

The PEG 400, Tween 80, and active ingredient were added slowly to solution B, and the mixture was kept under stirring until a homogeneous solution was obtained. At the end of the process, solution A, and the dispersion obtained was stirred for a further 30 minutes.

The resulting polymeric system was left to stand, up to complete removal of any air that might have been englobed therein, before being used.

| | |
|---|---|
| Backing layer | PVC |
| Protective film | siliconized paper |

Spreading on the backing layer and drying of the matrix were performed with a Matis spreading machine - Model: LTE-S

| | |
|---|---|
| Rate of spreading | 1m/min |
| Drying time | 15 min |
| Drying temperature: Distance between knife and protective film | 50°C 300 µm |

At the end of the drying process, the matrix dried on the backing layer was made to adhere to the protective film.

### EXAMPLE 11

| Composition of the polymeric system used for the preparation of the matrix | |
|---|---|
| Nifedipine | 0.6 |
| Eudragit L | 3.75 |
| Water | 5.75 |
| Sodium hydroxide | 0.675 |
| Water | 6.75 |
| PEG 400 | 5.625 |
| Glycerine | 2.45 |
| PVP | 1.88 |
| Methanol | 7.52 |

### Preparation

### Solution A

Eudragit L 100 was dispersed in water under propeller stirring at a speed of 100 rpm for 10 minutes. A solution of sodium hydroxide was prepared apart.

The solution of sodium hydroxide was added as fast as possible to the suspension of Eudragit L 100, and the speed of the stirrer was increased gradually up to 200 rpm. After a homogeneous solution had been obtained (approximately 5 minutes), PEG 400 and glycerine were added. The resulting system was kept under stirring at the same speed for a further 15 minutes and then left to stand, up to complete removal of any air that might have been entrapped therein.

### Solution B

The PVP was added very slowly to the methanol and kept under stirring with a mechanical stirrer up to complete solubilization. Any methanol that might have evaporated was restored.

The active ingredient was added to solution B, and the mixture was kept under stirring until a homogeneous solution was obtained. At the end of the process, solution A was added, and the dispersion obtained was stirred for a further 30 minutes.

The resulting polymeric system was left to stand, up to complete removal of any air that might have been englobed therein, before being used.

| | |
|---|---|
| Backing layer | PVC |
| Protective film | siliconized paper |

Spreading on the backing layer and drying of the matrix were performed with a Matis spreading machine - Model: LTE-S

| | |
|---|---|
| Rate of spreading | 1m/min |
| Drying time | 15 min |
| Drying temperature | 50°C |
| Distance between knife and protective film | 300 µm |

At the end of the drying process, the matrix dried on the backing layer was made to adhere to the protective film.

### EXAMPLE 12

| Composition of the polymeric system used for the preparation of the matrix | |
|---|---|
| Fentanyl | 1.56 |
| Eudragit L | 2.25 |
| Water | 3.45 |
| Sodium hydroxide | 0.405 |
| Water | 4.05 |
| PEG 400 | 3.375 |
| Glycerine | 1.47 |
| PVP | 1.34 |
| Water | 7.56 |

### Preparation

### Solution A

Eudragit L 100 was dispersed in water under mechanical stirring at a speed of 100 rpm for 10 minutes. A solution of sodium hydroxide was prepared apart.

The solution of sodium hydroxide was added as fast as possible to the suspension of Eudragit L 100, and the speed of the stirrer was increased gradually up to 200 rpm. After a homogeneous solution had been obtained (approximately 5 minutes), PEG 400 and glycerine were added. The resulting system was kept under stirring at the same speed for a further 15 minutes and then left to stand, up to complete removal of any air that might have been entrapped therein.

### Solution B

The PVP was added very slowly to the water preheated at 50°C and kept under stirring with a mechanical stirrer up to complete solubilization. Any water that might have evaporated was restored.

The Fentanyl was added slowly to solution A, and the mixture was kept under stirring until a homogeneous solution was obtained. At the end of the process, solution B was added, and the dispersion obtained was stirred for a further 30 minutes.

The resulting polymeric system was left to stand, up to complete removal of any air that might have been englobed therein, before being used.

| | |
|---|---|
| Backing layer | PVC |
| Protective film | siliconized paper |

Spreading on thebacking layer and drying of the matrix were performed with a Matis spreading machine - Model: LTE-S

| | |
|---|---|
| Rate of spreading | 1m/min |
| Drying time | 15 min |
| Drying temperature | 50°C |
| Distance between knife and protective film | 300 µm |

At the end of the drying process, the matrix dried on thebacking layer was made to adhere to the protective film.

### EXAMPLE 13

| Composition of the polymeric system used for the preparation of the matrix | |
|---|---|
| Thiocolchicoside | 1.56 |
| Eudragit L | 2.25 |
| Water | 3.45 |
| Sodium hydroxide | 0.405 |
| Water | 4.05 |
| PEG 400 | 3.375 |
| Glycerine | 1.47 |
| PVP | 1.34 |
| Ethanol | 7.56 |

### Preparation

### Solution A

Eudragit L 100 was dispersed in water preheated at 50°C under mechanical stirring at a speed of 100 rpm for 10 minutes. A solution of sodium hydroxide was prepared apart.

The solution of sodium hydroxide was added as fast as possible to the suspension of Eudragit L 100, and the speed of the stirrer was increased gradually up to 200 rpm. After a homogeneous solution had been obtained (approximately 5 minutes), PEG 400 and glycerine were added. The resulting system was kept under stirring at the same speed for a further 15 minutes and then left to stand, up to complete removal of any air that might have been entrapped therein.

### Solution B

The PVP was added very slowly to the ethanol and kept under stirring with a mechanical stirrer up to complete solubilization. Any ethanol that might have evaporated was restored.

The thiocolchicoside was added slowly to solution A, and the mixture was kept under stirring until a homogeneous solution was obtained. At the end of the process, solution B was added, and the dispersion obtained was stirred for a further 30 minutes.

The resulting polymeric system was left to stand, up to complete removal of any air that might have been englobed therein, before being used.

| | |
|---|---|
| Support | PVC |
| Protective film | siliconized paper |

Spreading on the backing layer and drying of the matrix were performed with a Matis spreading machine - Model: LTE-S

| | |
|---|---|
| Rate of spreading | 1m/min |
| Drying time | 15 min |
| Drying temperature | 50°C |
| Distance between knife and protective film | 300 µm |

At the end of the drying process, the matrix dried on thebacking layer was made to adhere to the protective film.

### EXAMPLE 14

| Composition of the polymeric systems used for the preparation of the matrices | | |
|---|---|---|
| Components | Form. No. 1 | Form. No. 2 |
| Lidocaine | 0.8 | 0.8 |
| Eudragit L 100 | | 4.5 |
| Eudragit L 100-55 | 4.5 | |
| Water | 6.9 | 6.9 |
| Sodium hydroxide | | 0.81 |
| Potassium hydroxide | 0.81 | |
| Water | 8.1 | 8.1 |
| PEG 400 | 6.75 | 6.75 |
| Glycerine | 2.94 | 2.94 |
| PVP | 2.25 | 2.25 |
| Ethanol | 9 | 9 |

### Preparation

### Solution A

Eudragit L 100 or L 100-55 was dispersed in water under mechanical stirring at a speed of 100 rpm for 10 minutes. A solution of sodium hydroxide or potassium hydroxide was prepared apart

The solution of sodium hydroxide or potassium hydroxide was added as fast as possible to the suspension of Eudragit L 100 or L 100-55, and the speed of the stirrer was increased gradually up to 200 rpm. After a homogeneous solution had been obtained (approximately 5 minutes), PEG 400 and glycerine were added. The resulting system was kept under stirring at the same speed for a further 15 minutes and then left to stand, up to complete removal of any air that might have been entrapped therein.

### Solution B

The PVP was added very slowly to the ethanol and kept under stirring with a mechanical stirrer up to complete solubilization. Any ethanol that might have evaporated was restored.

The active ingredient was added slowly to solution B, and the mixture was kept under stirring until a homogeneous solution was obtained. At the end of the process, solution A was added, and the dispersion obtained was stirred for a further 30 minutes.

The resulting polymeric system was left to stand, up to complete removal of any air that might have been englobed therein, before being used.

| | |
|---|---|
| Backing layer | PVC |
| Protective film | siliconized paper |

Spreading on thebacking layer and drying of the matrix were performed with a Matis spreading machine - Model: LTE-S

| | |
|---|---|
| Rate of spreading | 1 m/min |
| Drying time | 15 min |
| Drying temperature | 50°C |
| Distance between knife and protective film | 300 µm |

At the end of the drying process, the matrix dried on thebacking layer was made to adhere to the protective film.

### EXAMPLE 15

| Composition of the polymeric systems used for the preparation of the matrix | | | | | | | | |
|---|---|---|---|---|---|---|---|---|
| Components | Form No. 1 | Form No. 2 | Form No. 3 | Form No. 4 | Form No. 5 | Form No. 6 | Form No. 7 | Form No. 8 |
| Sodium | 2.2 | 3.22 | 5.4 | 5.03 | 2.15 | 2.5 | 2.5 | |
| Diclofenac epolamine | | | | | | | | 2.5 |
| Eudragit L 100 | 4.51 | 8.90 | 9.01 | 8.32 | 4.16 | | | |
| Eudr. L 100-55 | | | | | | 4.13 | 4.16 | 4.16 |
| Water | 9.59 | 18.89 | 19.18 | 23.5 | 11.71 | 12.01 | 9.86 | 9.86 |
| Sodium hydroxide | 0.60 | 1.19 | 1.20 | 1.11 | 0.56 | | | |
| Potassium hydroxide | | | | | | 0.55 | 0.77 | 0.77 |
| Water | 5.4 | 10.71 | 10.80 | 9.99 | 5.04 | 4.95 | 6.93 | 6.93 |
| PEG 400 | 6.76 | 15.82 | 13.52 | 9.33 | 4.67 | 6.24 | 6.19 | 6.19 |
| Glycerine | 2.94 | 5.81 | 5.89 | 5.43 | 2.72 | 2.72 | 2.70 | 2.70 |
| PVP | 1.8 | 3.57 | 3.6 | 3.32 | 1.66 | 1.35 | 1.35 | 1.35 |
| Ethanol | 8.65 | 18.91 | 17.7 | 16.13 | 8.06 | 6.9 | 6.9 | 6.9 |
| Succinic acid | 0.4 | 0.8 | 0.8 | 0.74 | 0.37 | 0.37 | 0.37 | 0.37 |
| Polyhydroxylated castor oil | 2 | 3.96 | 6.8 | 6.28 | | 3.15 | 3.15 | 3.15 |
| Span 80 | 0.2 | 0.39 | 0.41 | 0.38 | 0.19 | 0.17 | 0.17 | 0.17 |
| Isopropyl Myristate | 0.6 | 0.39 | 1.2 | 1.1 | 0.55 | 0.55 | 0.55 | 0.55 |
| Propylene glycol | | 1.18 | 6.7 | 9.34 | 4.67 | 3.1 | 3.1 | 3.1 |

### Preparation

### Solution A

Eudragit L 100 or L 100-55 was dispersed in water under propeller stirring at a speed of 100 rpm for 10 minutes. A solution of sodium hydroxide or potassium hydroxide was prepared apart.

The solution of sodium hydroxide or potassium hydroxide was added as fast as possible to the suspension of Eudragit L 100 or L 100-55, and the speed of the stirrer was increased gradually up to 200 rpm. After a homogeneous solution had been obtained (approximately 5 minutes), PEG 400 and glycerine were added.

The resulting system was kept under stirring at the same speed for a further 15 minutes and then left to stand, up to complete removal of any air that might have been entrapped therein.

### Solution B

The PVP was added very slowly to the ethanol and kept under stirring with a mechanical stirrer up to complete solubilization. Any ethanol that might have evaporated was restored.

The following were added in order to solution B: succinic acid, polyhydroxylated castor oil, SPAN 80, isopropyl myristate, propylene glycol, and active principle. The mixture was kept under stirring until a homogeneous solution was obtained. At the end of the process, solution A was added, and the dispersion obtained was stirred for a further 30 minutes.

The resulting polymeric system was left to stand, up to complete removal of any air that might have been englobed therein, before being used.

| | |
|---|---|
| Backing layer | PVC |
| Protective film | siliconized paper |

Spreading on the backing layer and drying of the matrix were performed with a Matis spreading machine - Model: LTE-S

| | |
|---|---|
| Rate of spreading | 1m/min |
| Drying time | 15 min |
| Drying temperature | 50°C |
| Distance between knife and protective film | 300 µm |

At the end of the drying process, the matrix dried on the backing layer was made to adhere to the protective film.

### EXAMPLE 16

| Composition of the polymeric systems used for the preparation of the matrices | | | | |
|---|---|---|---|---|
| | Form. No. 1 | Form. No. 2 | Form. No. 3 | Form. No. 4 |
| Ketoprofen | 1.56 | | | |
| Fentanyl | | 1.56 | | |
| Clonidine | | | 1.56 | |
| Thiocolchicoside | | | | 1.56 |
| Eudragit E | 12.6 | 12.6 | 12.6 | 12.6 |
| Water | 59.94 | 59.94 | 59.94 | 59.94 |
| Lauric acid | 7.56 | 7.56 | 7.56 | 7.56 |
| Adipic acid | 1.53 | 1.53 | 1.53 | 1.53 |
| Glycerine | 8.37 | 8.37 | 8.37 | 8.37 |
| PVP | 6.3 | 6.3 | 6.3 | 6.3 |
| Water | 25.2 | 25.2 | 25.2 | |
| Ethanol | | | | 25.2 |

### Preparation

### Solution A

Eudragit E, lauric acid and adipic acid were dispersed in water at 80°C under vacuum conditions and under mechanical stirring at a speed of 100 rpm for 60 minutes. The temperature was then brought down to 60°C, and glycerine was added. The resulting system was kept under stirring at the same speed for a further 30 minutes and then left to stand, up to complete removal of any air that might have been entrapped therein.

### Solution B

The PVP was added very slowly to the water or ethanol and kept under stirring with a mechanical stirrer up to complete solubilization. Any water or ethanol that might have evaporated was restored.

Solutions A and B and the active ingredient were mixed at room temperature using a mechanical stirrer until a homogeneous solution was obtained.

| | |
|---|---|
| Backing layer | PVC |
| Protective film | siliconized paper |

Spreading on the Backing layer and drying of the matrix were performed with a Matis spreading machine - Model: LTE-S

| | |
|---|---|
| Rate of spreading | 1m/min |
| Drying time | 15 min |
| Drying temperature | 50°C |
| Distance between knife and protective film | 300 µm |

At the end of the drying process, the matrix dried on the Backing layer was made to adhere to the protective film.

### PEEL-STRENGTH TEST

A number of examples are provided in what follows regarding determination of the peel strength of the patches prepared.

Determination of the peel strength was in accordance with the PSTC 1 method.

The analysis was carried out one week after preparation of the patches in order to enable stabilization of the matrix.

The test specimens, sized 2.5 x 20 cm, were applied, making sure that no air bubbles were formed, in the centre of the clean surface of the plate. When the patches were applied, care was taken not to exert an excessive force on the surface of contact, so as not to adversely affect the subsequent measurements. Then a constant strength of 20 N/cm was exerted by rolling a 5-kg roller three times in the direction of the length of the patch. The specimens thus prepared were left to rest for 10 minutes at 20°C. Peel strength was determined by applying the plate to an electronic load cell. The test was carried out using either a polyethylene plate or a steel plate.

### Operating conditions:

- peel angle: 180°
- rate of tensile force: 300 mm/min

The peel strength is expressed in cN/cm; for each specimen, three determinations were made.

### RESULTS OBTAINED USING A POLYETHYLENE PLATE

| | |
|---|---|
| Ex. 7, Form. 1 | 5.2 ± 1.1 cN/cm |
| Ex. 7, Form. 2 | 57.8 ± 2.5 cN/cm |
| Ex. 7, Form. 3 | 88.8 ± 12.7 cN/cm |
| Ex. 7, Form. 4 | 66.8 ± 11.2 cN/cm |
| Ex. 7, Form. 5 | 29.7 ± 1.4 cN/cm |
| Ex. 7, Form. 6 | 11.7 ± 2.2 cN/cm |
| Ex. 7, Form. 7 | 17.8 ± 3.6 cN/cm |
| Ex. 7, Form. 8 | 1.8 ± 0.2 cN/cm |
| Ex. 7, Form. 9 | 51.6 ± 13.6 cN/cm |

### RESULTS OBTAINED USING A STAINLESS STEEL PLATE

| | |
|---|---|
| Ex. 1, Form. 1 | 183.0 ± 16.0 cN/cm |
| Ex. 15, Form. 2 | 146.7 ± 31.0 cN/cm |
| Ex. 15, Form. 3 | 137.0 ±11.1 cN/cm |
| Ex. 15, Form. 4 | 145.5 ± 16.5cN/cm |
| Ex. 15, Form. 5 | 175.0 ±11.1 cN/cm |
| Ex. 15, Form 6 | 86.8 ± 11.9 cN/cm |
| Ex. 2, Form. 3 | 932.0 ±13.0 cN/cm |
| Ex. 16, Form. 1 | 519.0 ±3.0 cN/cm |

### SHEAR-STRENGTH TEST

A number of examples are provided in what follows regarding determination of the slip strength of the patches prepared.

The test specimens, sized 2.5 x 1.75 cm, were applied on plates of lapped aluminium, making sure that no air bubbles were formed and taking care not to exert an excessive force on the surface of contact, so as not to adversely affect the subsequent measurements. Then a constant strength of 20 N/cm was exerted on the surface of the specimen adhered to the plate, by rolling a 2.5-kg roller over it three times.

The specimen prepared was placed In a special structure that enabled an inclination of the plates of 2° with respect to the vertical position, so that the rear part of the panel formed an angle of 178° with the free end part of the specimen, to which a weight of 500 g was applied.

The strength was measured in terms of time necessary for the entire surface to be detached from the plate by slipping.

### RESULTS

| | |
|---|---|
| Ex. 6, Form. 1 | 164.4 ± 13 min. |
| Ex. 6, Form. 2 | 49.8 ± 1.6 min. |
| Ex. 6, Form. 3 | 4.0 ± 1.0 min. |
| Ex. 6, Form. 4 | 70.2 ± 8.9 min. |
| Ex. 6, Form. 5 | 20.2 ± 5.3 min. |
| Ex. 6, Form. 6 | 558.3 ± 4.4 min. |
| Ex. 6, Form. 7 | 96.6 ± 6 min. |
| Ex. 6, Form. 8 | 697.2 ± 42.4 min. |
| Ex. 6, Form. 9 | 267.9 ± 20.1 min. |
| Ex. 6, Form. 1 | 240.0 ± 0.3 min. |
| Ex. 2, Form. 3 | 4.2 ± 0.1 min. |
| Ex. 16, Form. 1 | 3.2 ± 0.3 min. |

### PERMEABILITY TESTS

A number of examples are provided in what follows regarding permeation of the active ingredients vehicled, also in comparison with products already available on the market.

The tests for permeability through the skin were carried out using a modified Franz diffusion cell.

As compared to the original model, the Franz cell used underwent a number of modifications. These consisted in the modification of the diameter of the receiving chamber, which was kept constant and equal to the maximum diameter in order to improve efficiency of mixing of the solution.

All the tests were carried out using human skin obtained by surgical operation. The layer of epidermis was prepared by immersion of the skin in distilled water at 60±1°C for one minute and subsequent mechanical separation of the dermis. The separated part was dehydrated in a dryer (25% RH) and kept in aluminium foil at-20°C up to the moment of use. This layer was then re-hydrated by immersion in a physiological solution (0.9% sodium chloride), at room temperature, for 16 hours, before being mounted on the Franz cells.

The patch specimen, which had a diameter of 18 mm, was made to adhere accurately and with a slight pressure exerted on the comeal layer immediately prior to being positioned on the cell in such a way that the part containing the active ingredient was facing the bottom chamber, in close contact with the receiving phase. The top chamber of the cell was set over the patch, and the two parts were isolated by using a teflon tape and parafilm® . The aim was to limit the evaporation of the receiving phase, and the two parts were held tightly together using a metal vice. The samples were taken from the sampling door positioned half-way up the cell, using 1-ml syringes. After each sampling, the receiving phase was re-integrated up to the same quantity. The sink conditions were maintained throughout the experiment. All the tests were conducted under a vertical laminar-flow hood.

### A) PERMEABILITY TEST OF FORMULATIONS NO. 1, NO. 3, NO. 6 OF EXAMPLE 8

### Operating conditions:

- temperature: 32±0.5°C
- area of release of patch: 0.636 cm²
- receiving phase: phosphate buffer solution at pH 7.4, degassed and sterilized by filtration with a 0.22-µm filter, with water having a degree of purity for HPLC.

Streptomycin sulphate was added to this solution as microbicidal agent.
- volume of receiving phase: approx. 5 ml exactly measured for each cell
- rate of stirring: maximum allowed by apparatus
- volume sampled: 200 µl
- The samples were taken at pre-set intervals.

### Result

| **EXAMPLE 8** | |
|---|---|
| Time | Form. No. 3 (µg/cm² of Ibuprofen) |
| 1 | 26.97 |
| 2 | 44.66 |
| 3 | 67.82 |
| 4 | 89.27 |
| 5 | 105.62 |
| 6 | 120.92 |
| 7 | 139.99 |
| 8 | 155.13 |
| 24 | 384.64 |

### B) PERMEABILITY TEST OF FORMULATION NO. 1 OF EXAMPLE 9

### Operating conditions:

- temperature: 32±0.5°C
- area of release of patch: 0.639 cm²
- receiving phase: phosphate buffer solution at pH 7.4, degassed and sterilised by filtration with a 0.22-µm filter, with water having a degree of purity for HPLC.

Streptomycin sulphate was added to this solution as microbicidal agent.
- volume of receiving phase: 5 ml exactly measured for each cell
- rate of stirring: maximum allowed by apparatus
- volume sampled: 200 µl
- The samples were taken at pre-set Intervals.

### Result

| | **EXAMPLE 9** | |
|---|---|---|
| Time | **Form. No. 1** (µg/cm² of Pyroxicam) | **Dexicam® gel** (µg/cm² of pyroxicam) |
| 1 | 1.28 | 1.49 |
| 3 | 1.45 | 1.64 |
| 5 | 1.70 | 1.84 |
| 7 | 2.05 | 2.00 |
| 24 | 3.98 | 3.84 |

### C) PERMEABILITY TEST OF FORMULATIONS NO. 3, NO. 4, NO. 5, NO.6, NO. 7 OF EXAMPLE 15

### Operating conditions:

- temperature: 32±0.5°C
- area of release of patch: 0.636 cm²
- receiving phase: physiological solution degassed and sterilized by filtration with a 0.22-µm filter, with water having a degree of purity for HPLC. Sodium azide was added to this solution as microbicldal agent.
- volume of receiving phase: 5 ml exactly measured for each cell
- rate of stirring: maximum allowed by apparatus
- volume sampled: 200 µl
- The samples were taken at pre-set intervals.

### Result

| | **EXAMPLE 15** | | | | | |
|---|---|---|---|---|---|---|
| Time | Form. No. 3 (µg/cm² of Diclofenac) | Form. No. 4 (µg/cm² of Diclofenac) | Form. No. 5 (µg/cm² of Diclofenac) | Form. No.6 (µg/cm² of Diclofenac) | Form. No.7 (µg/cm² of Diclofenac) | Dicloreum® Tissugel (µg/cm² of Diclofenac) |
| 1 | 1.84 | 0.92 | 0.51 | 1.10 | 0.98 | 0.30 |
| 3 | 2.72 | 1.06 | 0.54 | 1.37 | 1.83 | 0.94 |
| 6 | 3.79 | 1.48 | 0.91 | 2.15 | 3.36 | 2.27 |
| 8 | 4.45 | 1.84 | 1.41 | 2.80 | 4.60 | 3.16 |
| 24 | 14.50 | 8.21 | 14.18 | 12.42 | 18.26 | 11.48 |

### D) PERMEABILITY TEST OF FORM NO. 1, NO. 4 OF EXAMPLE 16

### Operating conditions:

- temperature: 32±0.5°C
- area of release of patch: 0.636 cm²
- receiving phase: phosphate buffer solution at pH 7.4, degassed and sterilized by filtration with a 0.22-µm filter, with water having a degree of purity for HPLC.

Streptomycin sulphate was added to this solution as microbicidal agent
- volume of receiving phase: 5 ml exactly measured for each cell
- rate of stirring: maximum allowed by apparatus
- volume sampled: 200 µl
- The samples were taken at pre-set Intervals.

### Result

| | **EXAMPLE 16** | | |
|---|---|---|---|
| Time | Form No. 1 µg/cm² of Ketoprofen) | Form No. 4 (µg/cm² of thiocolchicoside) | Muscoril® (µg/cm² of thiocolchicoside) |
| 1 | 0 | . 0 | 0.72 |
| 3 | 0 | 0.49 | 0.83 |
| 5 | 0.24 | 0.58 | 0.86 |
| 7 | 1.64 | 0.89 | 0.97 |
| 24 | 5.55 | 3.2 | 1.8 |

## Claims

1. A patch for local and transdermal administration of active ingredients having at least one anionic or electron-attracting group and pharmaceutically acceptable salts thereof comprising:
a) a prevalently aqueous polymeric matrix containing said active ingredient, whose polymer is selected from the group consisting of:
a-1) an anionic copolymer of methacrylic acid with a methacrylate ester with a C₁-C₁₀ linear or branched alcohol, or
a-2) a cationic copolymer of methacrylic acid with a C₁-C₁₀ linear or branched alcohol containing a tertiary aminic group, and a neutral methacrylate ester with a C₁-C₁₀ linear or branched alcohol,
said polymeric matrix further comprising:
a3) 30-100% by weight based on the co-polymer weight of a polyvinylpyrrolidone having an average molecular weight ranging from 300,000 to 1,350,000.
a4) a plasticizer and optionally the following components:
a5) enhancers,
a6) non ionic and anionic surfactants
a7) a hydroxide of an alkali or an earth alkali, in case in said matrix the anionic copolymer (a1) is contained.
b) a backing layer onto which the matrix (a) is adhered to,
c) a protective film removable at the moment of use, placed on said matrix (a).

2. A patch according to claim 1, wherein the anionic copolymer of type (a1) or cationic copolymer of type (a2) have an average molecular weight ranging from 80,000 to 500,000.

3. The patch according to anyone of claims 1-2, wherein the anionic copolymer of type (a1) or cationic copolymer of type (a2) have an average molecular weight from 100,000 to 300,000.

4. The patch according to anyone of claims 1 - 3 wherein the copolymer (a1) is selected from the group consisting of the copolymer of methacrylic acid and methylmethacrylate and the copolymer of methacrylic acid and ethylmethacrylate.

5. The patch according to anyone of claims 1-3, wherein the copolymer of type (a2) is selected from the group consisting of: the copolymer of dimethylaminoethylmethacrylate with methylmethacrylate, the copolymer of dimethylaminoethylmethacrylate with butylmethacrylate, and mixtures thereof.

6. The patch according to claim 4 wherein methacrylic acid -methylmethacrylate copolymer is used **characterised by** having a ratio of free carboxylic groups/ester groups being approximately 1:1 and an average molecular weight of 135,000.

7. The patch according to claim 4 wherein methacrylic add -ethylmethacrylate copolymer is used **characterised by** having the ratio of free carboxylic acid/ester groups is 1/1, and the average molecular weight of said copolymer is 250,000.

8. The patch according to claim 5 wherein a mixture of copolymer dimethylaminoethylmethacrylate - methylmethacrylate and copolymer dimethylaminoethylmethacrylate ― butylmethacrylate is used having an average molecular weight of 150,000.

9. The patch according to anyone of claims 1-8 wherein polyvinylpyrrolidone is used having an average molecular weight of: 335,000.

10. The patch according to anyone of claims 1-8 wherein polyvinylpyrrolidone is used having an average molecular weight of: 1,100,000.

11. The patch according to anyone of claims 1-10 wherein the plasticizer is selected from the group consisting of acetyltributyl citrate, tributyl citrate, glycerine, propylene glycol, polyethylene glycols (PEGS) of varying molecular weight, phthalates and triethyl citrate.

12. The patch according to anyone of claims 1-11, wherein the active ingredients containing anionic groups or an electronattracting group is selected from the group consisting of a non steroidal antiinflammatory, a muscle relaxant, antihypertensive, analgesic, local anaesthetic, and antianginal drug.

13. The patch according to anyone of claim 1-12, wherein the enhancers are selected from the group consisting of diethylenglycolmonoethylether, propylene glycol, polyethylene glycols, polyhydroxylated castor oil, unsaturated and saturated adds and esters thereof, polysorbates, polyoxyethylenalcohols and terpenes.

14. The patch according to anyone of claims 1-13, wherein the backing layer (b). consists of woven fabric, non-woven fabric, polymeric film and foaming material.

15. The patch according to anyone of claims 1-14, containing from 1 to 15 wt% of active ingredient with respect to the matrix weight

16. The patch according to Claim 15 containing from 2 to 6 wt% of active ingredient with respect to the matrix weight.

17. The patch according to anyone of claims 1-20 in which the plasticizing agent is contained in an amount of 5-200 wt% with respect to the copolymer (a1) or (a2) weight.

18. The patch according to any one of claims 1-17, wherein the surfactant is contained in an amount of 0.1-100 wt% with respect to the copolymer (a1) or (a2) weight.

19. The patch according to anyone of claims 1-18, wherein the enhancers are contained in an amount of 1-150 wt% with respect to the copolymer(a1) or (a2) weight.

20. A polymeric matrix containing an active ingredient having an anionic or an electron attracting group, whose polymer Is selected from the group consisting of:
a-1) an anionic copolymer of methacrylic acid with a methacrylate ester with a C₁-C₁₀ linear or branched alcohol, or
a-2) a cationic copolymer of methacrylic acid with a C₁-C₁₀ linear or branched alcohol containing a tertiary aminic group, and a neutral methacrylate ester with a C₁-C₁₀ linear or branched alcohol,
said polymeric matrix further comprising:
a3) 30-100% by weight based on the co-polymer weight of a polyvinylpyrrolidone having an average molecular weight ranging from 300,000 to 1,350,000.
a4) a plasticizer and optionally the following components:
a5) enhancers,
a6) non ionic and anionic surfactants

21. A process for preparing the patch according to anyone of claims 1-19, comprising:
i) preparing a prevalently aqueous solution of the copolymer (a1) or (a2)
ii) adding the active ingredient to a prevalently aqueous solution of the copolymer (a1) or (a2) containing the plasticizer (a4) or alternatively to that of the polyvinylpyrrolidone (a3),
iii) spreading and drying the mixture coming from step (ii) onto the protective film (c)
adhering the matrix(a) with the protective film as obtained in step (iii), to the backing layer (b).

## Patentansprüche

1. Ein Pflaster für die lokale und transdermale Verabreichung von Wirkstoffen, die mindestens eine anionische oder eine Elektronen anziehende Gruppe und pharmazeutisch zulässige Salze davon besitzen und sich wie folgt zusammensetzen aus:
a. einer vorwiegend wässrigen polymeren Matrix, die besagte Wirkstoffe enthält, wobei deren Polymer aus der Gruppe ausgewählt wird, bestehend aus:
a-1) einem anionischen Copolymer der Methacrylsäure mit einem Methacrylatester mit einem linearen oder verzweigten C₁-C₁₀-Alkohol oder
a-2) einem kationischen Copolymer der Methacrylsäure mit einem linearen oder verzweigten C₁-C₁₀-Alkohol, der eine tertiäre Aminogruppe besitzt und einem neutralen Methacrylatester mit einem linearen oder verzweigten C₁-C₁₀-Alkohol, besagter polymerer Matrix, die weiterhin beinhaltet:
a3) 30-100 Gewichts% des Copolymergewichtes eines Polyvinylpyrrolidons mit einem durchschnittlichen Molekulargewicht im Bereich von 300.000 bis 1.350.000.
a4) einem Weichmacher und optional den folgenden Komponenten:
a5) Enhancer
a6) nicht ionische und anionische oberflächenaktive Substanzen
a7) einem Alkali- oder Erdalkalihydroxid, im Fall, dass die besagte Matrix das anionische Copolymer (a1) enthält.
b) einer Trägerschicht, auf welcher die Matrix (a) haftet,
c) einem Schutzfilm über der Matrix (a), der erst vor Gebrauch entfernt wird.

2. Ein Pflaster gemäß Anspruch 1, wobei das anionische Copolymer vom Typ (a1) oder das kationische Copolymer vom Typ (a2) jeweils ein durchschnittliches Molekulargewicht von 80.000 bis 500.000 aufweist.

3. Das Pflaster gemäß einem der Ansprüche von 1-2, wobei das anionische Copolymer vom Typ (a1) oder das kationische Copolymer vom Typ (a2) jeweils ein durchschnittliches Molekulargewicht 100.000 bis 300.000 aufweist

4. Das Pflaster gemäß einem der Ansprüche von 1-3, wobei das Copolymer (a1) aus der Gruppe ausgewählt wird, die das Copolymer der Methacrylsäure und Methylmethacrylat sowie das Copolymer der Methacrylsäure und das Ethylmethacrylat beinhaltet.

5. Das Pflaster gemäß einem der Ansprüche von 1-3, wobei das Copolymer vom Typ (a2) ausgewählt wird aus der Gruppe, bestehend aus: dem Copolymer des Dimethylaminoethylmethacrylat mit Methylmethacrylat, dem Copolymer von Dimethylaminoethylmethacrylat mit Butylmethacrylat und Mischungen daraus.

6. Das Pflaster gemäß Anspruch 4, wobei das Methacrylsäure-Methylmethacrylat-Copolymer eingesetzt wird, das charakterisiert ist durch ein Verhältnis von freien Carboxylsäure-/Estergruppen von annähernd 1:1 und einem durchschnittlichen Molekulargewicht von 135.000.

7. Das Pflaster gemäß Anspruch 4, wobei das Methacrylsäure-Ethylmethacrylat-Copolymer eingesetzt wird, das charakterisiert ist durch ein Verhältnis von freien Carboxylsäure-/Estergruppen von 1/1 und einem durchschnittlichen Molekulargewicht des besagten Copolymers von 250.000.

8. Das Pflaster gemäß Anspruch 5, wobei eine Mischung aus den Copolymeren Dimethylaminoethylmethacrylat-Methylmethacrylat und Dimethylaminoethylmethacrylat-Butylmethacrylat eingesetzt wird mit einem durchschnittlichen Molekulargewicht von 150.000.

9. Das Pflaster gemäß einem der Ansprüche von 1-8, wobei Polyvinylpyrrolidon eingesetzt wird mit einem durchschnittlichen Molekulargewicht von 335.000.

10. Das Pflaster gemäß einem der Ansprüche von 1-8, wobei Polyvinylpyrrolidon eingesetzt wird mit einem durchschnittlichen Molekulargewicht von 1.100.000.

11. Das Pflaster gemäß einem der Ansprüche von 1-10, wobei der Weichmacher ausgewählt wird aus der Gruppe, die wie folgt beinhaltet: Acetyltributylcitrat, Tributylcitrat, Glycerin, Propylenglykol, Polyethylenglykole (PEG) mit unterschiedlichem Molekulargewicht, Phthalate und Triethylcitrat.

12. Das Pflaster gemäß einem der Ansprüche von 1-11, wobei die Wirkstoffe anionische Gruppen oder eine Elektronen anziehende Gruppe enthalten und aus der Gruppe ausgewählt werden, die eine nicht steroidale antiinflammatorische Medikation, ein Muskelrelaxans, ein Antihypertensivum, ein Analgetikum, ein lokales Anästhetikum und eine antianginal wirkende Medikation enthält.

13. Das Pflaster gemäß einem. der Ansprüche von 1-12, wobei die Enhancer aus der Gruppe ausgewählt werden, bestehend aus: Diethylenglykolmonoethylether, Propylenglykol, Polyethylenglykole, polyhydroxyliertem Rizinusöl, ungesättigten und gesättigten Säuren und Ester davon, Polysorbate, Polyoxyethylenalkohole und Terpene.

14. Das Pflaster gemäß einem der Ansprüche von 1-13, wobei die Trägerschicht (b) aus einem gewobenen und einem nicht gewobenen Gewebe, einem polymeren Film und einem Schaumstoff besteht.

15. Das Pflaster gemäß einem der Ansprüche von 1-14, das mit Bezug auf das Gesamtgewicht der Matrix einen Anteil von 1-15 Gewichts% an Wirkstoff enthält.

16. Das Pflaster gemäß Anspruch 15, das mit Bezug auf das Gesamtgewicht der Matrix einen Anteil von 2-6 Gewichts% an Wirkstoff enthält.

17. Das Pflaster gemäß einem der Ansprüche von 1-20, bei dem der Weichmacher mit einem Anteil von 5-200 Gewichts% mit Bezug auf das Copolymergewicht (a1) oder (a2) enthalten ist.

18. Das Pflaster gemäß einem der Ansprüche von 1-17, bei dem die oberflächenaktive Substanz mit einem Anteil von 0,11-100 Gewichts% mit Bezug auf das Copolymergewicht (a1) oder (a2) enthalten ist.

19. Das Pflaster gemäß einem der Ansprüche von 1-18, bei dem die Enhancer mit einem Anteil von 1-150 Gewichts% mit Bezug auf das Copolymergewicht (a1) oder (a2) enthalten sind.

20. Eine polymere Matrix, die einen Wirkstoff mit einer anionischen oder Elektronen anziehenden Gruppe enthält, deren Polymer aus der Gruppe ausgewählt wird, die wie folgt enthält:
a-1) ein anionisches Coploymer der Methacrylsäure mit einem linearen oder verzweigten C₁-C₁₀-Alkohol, oder
a-2) ein kationisches Copolymer der Methacrylsäure mit einem linearen oder verzweigten C₁-C₁₀-Alkohol, der eine tertiäre Aminogruppe enthält und einen neutralen Methacrylatester mit einem linearen oder verzweigten C₁-C₁₀-Alkohol; die besagte polymere Matrix beinhaltet weiterhin:
a-3) 30-100 Gewichtsprozent in Bezug auf das Copolymergewicht eines Polyvinylpyrrolidons mit einem durchschnittlichen Molekulargewicht im Bereich von 300.000 bis 1.350.000.
a4) einen Weichmacher und optional die folgenden Komponenten:
a5) Enhancer
a6) nicht ionische und anionische oberflächenaktive Substanzen
21. Ein Prozess zur Herstellung eines Pflasters gemäß einem der Ansprüche von 1-19, der wie folgt umfasst:
I) Herstellen einer vorwiegend wässrigen Lösung des Copolymers (a1) oder (a2)
II) Wirkstoffzugabe zur überwiegend wässrigen Copolymerlösung (a1) oder (a2), die den Weichmacher (a4) enthält oder alternativ Zugabe zu Polyvinylpyrrolidon (a3),
III) Verteilen und Trocknen der Mischung aus Schritt (II) auf dem Schutzfilm (c) aufbringen der Matrix (a) mit dem wie in Schritt (III) erhaltenen Schutzfilm auf die Trägerschicht (b)

## Revendications

1. Timbre pour administration par voie locale et transdermique de principes actifs ayant au moins un groupe anionique ou électroattracteur et de leurs sels pharmaceutiquement acceptables, comprenant :
a) une matrice polymère aqueuse prédominante contenant ledit principe actif, dont le polymère est choisi dans le groupe constitué par :
a-1) un copolymère anionique d'acide méthacrylique avec un ester méthacrylique d'un alcool linéaire ou ramifié en C₁-C₁₀;
a-2) un copolymère cationique d'acide méthacrylique avec un alcool linéaire ou ramifié en C₁-C₁₀ contenant un groupe amine tertiaire, et un ester neutre méthacrylique d'un alcool linéaire ou ramifié en C₁-C₁₀ ;
ladite matrice polymère contenant en outre
a3) de 30 à 100% en poids par rapport au poids de copolymère de polyvinylpyrolidone ayant un poids moléculaire moyen allant de 300 000 à 1 350 000 ;
a4) un plastifiant et éventuellement les composés suivants,
a5) des activateurs,
a6) des tensioactifs non ioniques ou anioniques,
a7) un hydroxyde d'alcalin ou d'alcalino-terreux, lorsque ladite matrice contient le copolymère (a1) ;
b) une couche support à laquelle la matrice (a) adhère,
c) un film de protection qui peut être retiré lors de l'utilisation, placé sur la matrice (a).

2. Timbre selon la revendication 1, dans lequel le copolymère anionique du type (a1) ou le copolymère cationique du type (a2) ont un poids moléculaire moyen allant de 80 000 à 500 000.

3. Timbre selon l'une quelconque des revendications 1 ou 2, dans lequel le copolymère anionique du type (a1) ou le copolymère cationique du type (a2) ont un poids moléculaire moyen allant de 100 000 à 300 000.

4. Timbre selon l'une quelconque des revendications 1 à 3, dans lequel le copolymère (a1) est choisi dans le groupe constitué par le copolymère d'acide méthacrylique et de méthacrylate de méthyle, et le copolymère d'acide méthacrylique et de méthacrylate d'éthyle.

5. Timbre selon l'une quelconque des revendications 1 à 3, dans lequel le copolymère (a2) est choisi dans le groupe constitué par le copolymère du méthacrylate de diméthylaminoéthyle et de méthacrylate de méthyle, le copolymère de méthacrylate de diméthylaminoéthyle et de méthacrylate de butyle, et leurs mélanges.

6. Timbre selon la revendication 4, dans lequel le copolymère acide méthacrylique - méthacrylate de méthyle est utilisé, **caractérisé en ce que** le rapport groupes carboxyliques libres/groupes esters est d'environ 1/1 et le poids moléculaire moyen est de 135 000.

7. Timbre selon la revendication 4, dans lequel le copolymère acide méthacrylique - méthacrylate d'éthyle est utilisé, **caractérisé en ce que** le rapport groupes carboxyliques libres/groupes esters est d'environ 1/1, et le poids moléculaire moyen est de 250 000.

8. Timbre selon la revendication 5, dans lequel un mélange de copolymère méthacrylate de diméthylaminoéthyle-méthacrylate de méthyle et de copolymère méthacrylate de diméthylaminoéthyle-méthacrylate de butyle ayant un poids moléculaire moyen de 150 000 est utilisé.

9. Timbre selon l'une quelconque des revendications 1 à 8, dans lequel une polyvinylpyrolidone ayant un poids moléculaire moyen de 335 000 est utilisée.

10. Timbre selon l'une quelconque des revendications 1 à 8, dans lequel une polyvinylpyrolidone ayant un poids moléculaire moyen de 1 100 000 est utilisée.

11. Timbre selon l'une quelconque des revendications 1 à 10, dans lequel le plastifiant est choisi dans le groupe constitué par le citrate d'acétyltributyle, le citrate de tributyle, la glycérine, le propylène glycol, les polyéthylène glycols (PEGs) de différents poids moléculaires, les phtalates et le citrate de triéthyle.

12. Timbre selon l'une quelconque des revendications 1 à 11, dans lequel le principe actif contenant des groupes anioniques ou un groupe électroattracteur est choisi dans le groupe constitué par un anti-inflammatoire non stéroïdien, un relaxant musculaire, un antihypertension, un analgésique, un anesthésique local, et un médicament antiangineux.

13. Timbre selon l'une quelconque des revendications 1 à 12, dans lequel les activateurs sont choisis dans le groupe constitué par le diéthylèneglycolmonoéthyléther, le propylène glycol, les polyéthylène glycols, l'huile de ricin polyhydroxylée, des acides insaturés et saturés et leurs esters, les polysorbates, les polyoxyéthylène alcools et les terpènes.

14. Timbre selon l'une quelconque des revendications 1 à 13, dans lequel la couche support (b) est constituée par un textile tissé, un textile non tissé, un film polymère ou une mousse.

15. Timbre selon l'une quelconque des revendications 1 à 14, contenant de 1 à 15% en poids de principe actif par rapport au poids de la matrice.

16. Timbre selon la revendication 15, contenant de 2 à 6% en poids de principe actif par rapport au poids de la matrice.

17. Timbre selon l'une quelconque des revendications 1 à 20, dans lequel l'agent plastifiant représente de 5 à 200% en poids par rapport au poids de copolymère (a1) ou (a2).

18. Timbre selon l'une quelconque des revendications 1 à 17, dans lequel l'agent tensioactif représente de 0,1 à 100% en poids par rapport au poids de copolymère (a1) ou (a2).

19. Timbre selon l'une quelconque des revendications 1 à 18, dans lequel les activateurs représentent de 1 à 150% en poids par rapport au poids de copolymère (a1) ou (a2).

20. Matrice polymère contenant un principe actif ayant un groupe anionique ou un groupe électroattracteur, dont le polymère est choisi dans le groupe constitué par :
a-1) un copolymère anionique d'acide méthacrylique avec un ester méthacrylique d'un alcool linéaire ou ramifié en C₁-C₁₀ ;
a-2) un copolymère cationique d'acide méthacrylique avec un alcool linéaire ou ramifié en C₁-C₁₀ contenant un groupe amine tertiaire, et un ester neutre méthacrylique d'un alcool linéaire ou ramifié en C₁-C₁₀ ;
ladite matrice polymère contenant en outre
a3) de 30 à 100% en poids par rapport au poids de copolymère de polyvinylpyrolidone ayant un poids moléculaire moyen allant de 300 000 à 1 350 000 ;
a4) un plastifiant et éventuellement les composés suivants,
a5) des activateurs,
a6) des tensioactifs non ioniques ou anioniques.

21. Procédé pour la préparation d'un timbre selon l'une quelconque des revendications 1 à 19, dans lequel :
i) on prépare une solution aqueuse prédominante de copolymère (a1) ou (a2) ;
ii) on ajoute le principe actif à la solution aqueuse préformée de copolymère (a1) ou (a2) contenant le plastifiant (a4) ou, en variante, à la solution de polyvinylpyrolidone (a3) ;
iii) on étale le mélange obtenu à l'étape (ii) sur le film protecteur (c) et on le sèche,
faisant adhérer la matrice (a) au film protecteur obtenu dans l'étape (iii) à la couche support (b).
